# EUROPEAN PATENT SPECIFICATION

(11) **EP 1 317 547 B1**
(45) Date of publication and mention of the grant of the patent: **25.01.2012**
(21) Application number: 01960723.3
(22) Date of filing: 05.09.2001
(51) Int. Cl.: C12N 15/54, C12N 15/74, C12N 1/21, C12N 9/12, C12P 13/04, C12P 13/08, C12Q 1/68

(54) **ISOLATION AND SEQUENCING OF THE PKNB GENE OF C. GLUTAMICUM**
ISOLIERUNG UND SEQUENZIERUNG VOM GEN PKNB AUS C. GLUTAMICUM
ISOLEMENT ET SEQUEN AGE DU GENE PKNB DE I C. GLUTAMICUM /I

(30) Priority: 12.09.2000 DE 10044912; 25.04.2001 DE 10120095
(43) Date of publication of application: 11.06.2003
(73) Proprietor: Evonik Degussa GmbH, 45128 Essen (DE)
(72) Inventor: BATHE, Brigitte, 33154 Salzkotten (DE); HANS, Stephan, 49078 Osnabrück (DE); FARWICK, Mike, 33615 Bielefeld (DE); HERMANN, Thomas, 33739 Bielefeld (DE)
(86) International application number: PCT/EP2001/010211
(87) International publication number: WO 2002/022828

(56) References cited:
- EP-A- 1 029 919
- EP-A- 1 108 790
- DATABASE SWALL [Online] EBI; 1 October 1996 (1996-10-01) "M. leprae PknB" XP002185694
- CREMER J ET AL: "CONTROL OF THE LYSINE BIOSYNTHESIS SEQUENCE IN CORYNEBACTERIUM GLUTAMICUM AS ANALYZED BY OVEREXPRESSION OF THE INDIVIDUAL CORRESPONDING GENES" APPLIED AND ENVIRONMENTAL MICROBIOLOGY, WASHINGTON,DC, US, vol. 57, no. 6, 1 June 1991 (1991-06-01), pages 1746-1752, XP000616281 ISSN: 0099-2240

## Description

### Field of the Invention

The invention provides nucleotide sequences from corynebacteria coding for the pknB gene and a fermentation process for the preparation of L-Lysin using bacteria in which the endogeny pknB gene is amplified.

### State of the Art

L-Amino acids, especially L-lysine, are used in human medicine, in the pharmaceutical industry, in the food industry and very especially in animal nutrition.

It is known that amino acids are prepared by the fermentation of strains of corynebacteria, especially Corynebacterium glutamicum. Because of their great importance, attempts are constantly being made to improve the preparative processes. Improvements to the processes may relate to measures involving the fermentation technology, e.g. stirring and oxygen supply, or the composition of the nutrient media, e.g. the sugar concentration during fermentation, or the work-up to the product form, e.g. by ion exchange chromatography, or the intrinsic productivity characteristics of the microorganism itself.

The productivity characteristics of these microorganisms are improved by using methods of mutagenesis, selection and mutant choice to give strains which are resistant to antimetabolites or auxotrophic for metabolites important in regulation, and produce amino acids.

Methods of recombinant DNA technology have also been used for some years to improve L-amino acid-producing strains of Corynebacterium by amplifying individual amino acid biosynthesis genes and studying the effect on amino acid production.

### Object of the Invention

The object which the inventors set themselves was to provide novel measures for improving the preparation of L-Lysin by fermentation.

This object is achieved according to claims 1 to 12 .

### Summary of the Invention

When L-amino acids or amino acids are mentioned hereafter, they are understood as meaning one or more amino acids, including their salts, selected from the group comprising L-asparagine, L-threonine, L-serine, L-glutamate, L-glycine, L-alanine, L-cysteine, L-valine, L-methionine, L-isoleucine, L-leucine, L-tyrosine, L-phenylalanine, L-histidine, L-lysine, L-tryptophan and L-arginine. L-Lysine is particularly preferred.

The invention provides an isolated polynucleotide from corynebacteria selected from the group comprising:
a) a polynucleotide containing the nucleotide sequence of SEQ ID NO:1
b) a polynucleotide which is complementary to the polynucleotides of a)

The invention also provides the above-mentioned polynucleotide, which is a recombinant DNA replicatable in corynebacteria.

The invention also provides:
a replicatable polynucleotide, especially DNA, containing the nucleotide sequence as shown in SEQ ID No. 1,
a vector containing the polynucleotide according to the invention, especially a shuttle vector or plasmid vector, and corynebacteria which contain the vector or in which the endogeny pknB gene is amplified.

The description further discloses polynucleotides consisting substantially of a polynucleotide sequence which are obtainable by screening, by means of hybridization, of an appropriate gene library of a corynebacterium, containing the complete gene or parts thereof, with a probe containing the sequence of the polynucleotide of the invention according to SEQ ID No. 1 or a fragment thereof, and by isolation of said polynucleotide sequence.

### Detailed Description of the Invention

As hybridization probes for RNA, cDNA and DNA, polynucleotides containing the sequences according to the invention are suitable for isolating the full length of nucleic acids, or polynucleotides or genes, coding for protein kinase B, or for isolating nucleic acids, or polynucleotides or genes, whose sequence exhibits a high degree of similarity to the sequence of the pknB gene. They are also suitable for incorporation into so-called arrays, micro-arrays or DNA chips for detecting and determining the corresponding polynucleotides.

Polynucleotides containing the sequences according to the invention are further suitable as primers for the preparation, by the polymerase chain reaction (PCR), of DNA of genes coding for protein kinase B.

Such oligonucleotides serving as probes or primers contain at least 25, 26, 27, 28, 29 or 30, preferably at least 20, 21, 22, 23 or 24 and very particularly preferably at least 15, 16, 17, 18 or 19 consecutive nucleotides. Oligonucleotides with a length of at least 31, 32, 33, 34, 35, 36, 37, 38, 39 or 40 or at least 41, 42, 43, 44, 45, 46, 47, 48, 49 or 50 nucleotides are also suitable. Oligonucleotides with a length of at least 100, 150, 200, 250 or 300 nucleotides may also be suitable.

"Isolated" means separated from its natural environment.

"Polynucleotide" refers in general to polyribonucleotides and polydeoxyribonucleotides, it being possible for the RNAs or DNAs in question to be unmodified or modified.

The polynucleotides according to the invention include a polynucleotide according to SEQ ID No. 1

"Polypeptides" are understood as meaning peptides or proteins containing two or more amino acids bonded via peptide links.

The invention further relates to a fermentation process for the preparation of L-Lysine, using corynebacteria which, in particular, already produce amino acids and in which the nucleotide sequences coding for the pknB gene are amplified and, in particular, overexpressed.

In this context the term "enhancement" describes the increase in the intracellular activity, in a microorganism, of one or more enzymes which are coded for by the appropriate DNA, for example by increasing the copy number of the gene(s) or allele(s), using a strong promoter or using a gene or allele coding for an appropriate enzyme with a high activity, and optionally combining these measures.

By enhancement measures, in particular over-expression, the activity or concentration of the corresponding protein is in general increased by at least 10%, 25%, 50%, 75%, 100%, 150%, 200%, 300%, 400% or 500%, up to a maximum of 1000% or 2000%, based on the starting microorganism.

The microorganisms provided by the present invention can produce L-Lysin from glucose, sucrose, lactose, fructose, maltose, molasses, starch or cellulose or from glycerol and ethanol. Said microorganisms can be representatives of corynebacteria, especially of the genus Corynebacterium. The species Corynebacterium glutamicum may be mentioned in particular in the genus Corynebacterium, being known to those skilled in the art for its ability to produce L-amino acids.

The following known wild-type strains: .
Corynebacterium glutamicum ATCC13032
Corynebacterium acetoglutamicum ATCC15806
Corynebacterium acetoacidophilum ATCC13870
Corynebacterium thermoaminogenes FERM BP-1539
Corynebacterium melassecola ATCC17965
Brevibacterium flavum ATCC14067
Brevibacterium lactofermentum ATCC13869 and
Brevibacterium divaricatum ATCC14020
and L-Lysin -producing mutants or strains prepared therefrom, are particularly suitable strains of the genus Corynebacterium, especially of the species Corynebacterium glutamicum (C. glutamicum).

The novel pknB gene of C. glutamicum coding for the enzyme protein kinase B (EC 2.7.1.37) has been isolated.

The first step in isolating the pknB gene or other genes of C. glutamicum is to construct a gene library of this microorganism in Escherichia coli (E. coli). The construction of gene libraries is documented in generally well-known textbooks and manuals. Examples which may be mentioned are the textbook by Winnacker entitled From Genes to Clones, Introduction to Gene Technology (Verlag Chemie, Weinheim, Germany, 1990) or the manual by Sambrook et al. entitled Molecular Cloning, A Laboratory Manual (Cold Spring Harbor Laboratory Press, 1989). A very well-known gene library is that of the E. coli K-12 strain W3110, which was constructed by Kohara et al. (Cell 50, 495-508 (1987)) in λ vectors. Bathe et al. (Molecular and General Genetics 252, 255-265, 1996) describe a gene library of C. glutamicum ATCC13032, which was constructed using cosmid vector SuperCos I (Wahl et al., 1987, Proceedings of the National Academy of Sciences USA 84, 2160-2164) in the E. coli K-12 strain NM554 (Raleigh et al., 1988, Nucleic Acids Research 16, 1563-1575).

Börmann et al. (Molecular Microbiology 6(3), 317-326 (1992)) in turn describe a gene library of C. glutamicum ATCC13032 using cosmid pHC79 (Hohn and Collins, Gene 11, 291-298 (1980)).

A gene library of C. glutamicum in E. coli can also be constructed using plasmids like pBR322 (Bolivar, Life Sciences 25, 807-818 (1979)) or pUC9 (Vieira et al., 1982, Gene 19, 259-268). Restriction- and recombination-defective E. coli strains are particularly suitable as hosts, an example being the strain DH5αmcr, which has been described by Grant et al. (Proceedings of the National Academy of Sciences USA 87 (1990) 4645-4649). The long DNA fragments cloned with the aid of cosmids can then in turn be subcloned into common vectors suitable for sequencing, and subsequently sequenced, e.g. as described by Sanger et al. (Proceedings of the National Academy of Sciences of the United States of America 74, 5463-5467, 1977).

The DNA sequences obtained can then be examined with known algorithms or sequence analysis programs, e.g. that of Staden (Nucleic Acids Research 14, 217-232 (1986)), that of Marck (Nucleic Acids Research 16, 1829-1836 (1988)) or the GCG program of Butler (Methods of Biochemical Analysis 39, 74-97 (1998)).

The novel DNA sequence of C. glutamicum coding for the pknB gene was found and, as SEQ ID No. 1, forms part of the present invention.

Those skilled in the art will find instructions on the identification of DNA sequences by means of hybridization in the manual entitled "The DIG System User's Guide for Filter Hybridization" from Boehringer Mannheim GmbH (Mannheim, Germany, 1993) and in Liebl et al. (International Journal of Systematic Bacteriology (1991) 41, 255-260), inter alia. Hybridization takes place under stringent conditions; in other words, only hybrids for which the probe and the target sequence, i.e. the polynucleotides treated with the probe, are at least 70% identical are formed. It is known that the stringency of hybridization, including the washing steps, is influenced or determined by varying the buffer composition, the temperature and the salt concentration. The hybridization reaction is preferably carried out under relatively low stringency compared with the washing steps (Hybaid Hybridisation Guide, Hybaid Limited, Teddington, UK, 1996).

The hybridization reaction can be carried out for example using a 5x SSC buffer at a temperature of approx. 50°C - 68°C, it also being possible for probes to hybridize with polynucleotides which are less than 70% identical to the sequence of the probe. Such hybrids are less stable and are removed by washing under stringent conditions. This can be achieved for example by lowering the salt concentration to 2x SSC and subsequently to 0.5x *SSC* if necessary (The DIG System User's Guide for Filter Hybridization, Boehringer Mannheim, Mannheim, Germany, 1995), the temperature being adjusted to approx. 50°C - 68°C. It is possible to lower the salt concentration to 0.1x SSC if necessary. By raising the hybridization temperature in approx. 1 - 2°C steps from 50°C to 68°C, it is possible to isolate polynucleotide fragments which are e.g. at least 70%, at least 80% or at least 90% to 95% identical to the sequence of the probe used. Further instructions on hybridization are commercially available in the form of kits (e.g. DIG Easy Hyb from Roche Diagnostics GmbH, Mannheim, Germany, Catalog No. 1603558).

Those skilled in the art will find instructions on the amplification of DNA sequences with the aid of the polymerase chain reaction (PCR) in the manual by Gait entitled Oligonucleotide Synthesis: A Practical Approach (IRL Press, Oxford, UK, 1984) and in Newton and Graham: PCR (Spektrum Akademischer Verlag, Heidelberg, Germany, 1994), inter alia.

It has been found that, after overexpression of the pknB gene, the production of L-Lysin by corynebacteria is improved.

Overexpression can be achieved by increasing the copy number of the appropriate genes or mutating the promoter and regulatory region or the ribosome binding site located upstream from the structural gene. Expression cassettes incorporated upstream from the structural gene work in the same way. Inducible promoters additionally make it possible to increase the expression in the course of the production of amino acid by fermentation. Measures for prolonging the life of the mRNA also improve the expression. Furthermore, the enzyme activity is also enhanced by preventing the degradation of the enzyme protein. The genes or gene constructs can either be located in plasmids of variable copy number or integrated and amplified in the chromosome. Alternatively, it is also possible to achieve overexpression of the genes in question by changing the composition of the media and the culture technique.

Those skilled in the art will find relevant instructions in Martin et al. (Bio/Technology 5, 137-146 (1987)), Guerrero et al. (Gene 138, 35-41 (1994)), Tsuchiya and Morinaga (Bio/Technology 6, 428-430 (1988)), Eikmanns et al. (Gene 102, 93-98 (1991)), EP 0 472 869, US 4,601,893, Schwarzer and Pühler (Bio/Technology 9, 84-87 (1991)), Reinscheid et al. (Applied and Environmental Microbiology 60, 126-132 (1994)), LaBarre et al. (Journal of Bacteriology 175, 1001-1007 (1993)), WO 96/15246, Malumbres et al. (Gene 134, 15-24 (1993)), JP-A-10-229891, Jensen and Hammer (Biotechnology and Bioengineering 58, 191-195 (1998)) and Makrides (Microbiological Reviews 60, 512-538 (1996)), inter alia, and in well-known textbooks on genetics and molecular biology.

For amplification, the pknB gene according to the invention has been overexpressed for example with the aid of episomal plasmids. Suitable plasmids are those which are replicated in corynebacteria. Numerous known plasmid vectors, e.g. pZ1 (Menkel et al., Applied and Environmental Microbiology (1989) 64, 549-554), pEKEx1 (Eikmanns et al., Gene 102, 93-98 (1991)) or pHS2-1 (Sonnen et al., Gene 107, 69-74 (1991)), are based on cryptic plasmids pHM1519, pBL1 or pGA1. Other plasmid vectors, e.g. those based on pCG4 (US-A-4,489,160), pNG2 (Serwold-Davis et al., FEMS Microbiology Letters 66, 119-124 (1990)) or pAG1 (US-A-5,158,891), can be used in the same way.

Other suitable plasmid vectors are those which make it possible to use the gene amplification process by integration into the chromosome, as described for example by Reinscheid et al. (Applied and Environmental Microbiology 60, 126-132 (1994)) for the duplication or amplification of the hom-thrB operon. In this method the complete gene is cloned into a plasmid vector which can replicate in a host (typically E. coli), but not in C. glutamicum. Examples of suitable vectors are pSUP301 (Simon et al., Bio/Technology 1, 784-791 (1983)), pK18mob or pK19mob (Schäfer et al., Gene 145, 69-73 (1994)), pGEM-T (Promega Corporation, Madison, WI, USA), pCR2.1-TOPO (Shuman (1994), Journal of Biological Chemistry 269, 32678-84; US-A-5,487,993), pCR^{®}Blunt (Invitrogen, Groningen, The Netherlands; Bernard et al., Journal of Molecular Biology 234, 534-541 (1993)), pEM1 (Schrumpf et al., 1991, Journal of Bacteriology 173, 4510-4516) or pBGS8 (Spratt et al., 1986, Gene 41, 337-342). The plasmid vector containing the gene to be amplified is then transferred to the desired strain of C. glutamicum by conjugation or transformation. The method of conjugation is described for example in Schäfer et al. (Applied and Environmental Microbiology 60, 756-759 (1994)). Methods of transformation are described for example in Thierbach et al. (Applied Microbiology and Biotechnology 29, 356-362 (1988)), Dunican and Shivnan (Bio/Technology 7, 1067-1070 (1989)) and Tauch et al. (FEMS Microbiological Letters 123, 343-347 (1994)). After homologous recombination by means of a crossover event, the resulting strain contains at least two copies of the gene in question.

The microorganisms prepared according to the invention are also provided by the invention and can be cultivated for the production of L-Lysin continuously or discontinuously by the batch process, the fed batch process or the repeated fed batch process. A summary of known cultivation methods is described in the textbook by Chmiel (Bioprozesstechnik 1. Einführung in die Bioverfahrenstechnik (Bioprocess Technology 1. Introduction to Bioengineering) (Gustav Fischer Verlag, Stuttgart, 1991)) or in the textbook by Storhas (Bioreaktoren und periphere Einrichtungen (Bioreactors and Peripheral Equipment) (Vieweg Verlag, Brunswick/Wiesbaden, 1994)).

The culture medium to be used must appropriately meet the demands of the particular strains. Descriptions of culture media for various microorganisms can be found in "Manual of Methods for General Bacteriology" of the American Society for Bacteriology (Washington DC, USA, 1981).

Carbon sources which can be used are sugars and carbohydrates, e.g. glucose, sucrose, lactose, fructose, maltose, molasses, starch and cellulose, oils and fats, e.g. soybean oil, sunflower oil, groundnut oil and coconut fat, fatty acids, e.g. palmitic acid, stearic acid and linoleic acid, alcohols, e.g. glycerol and ethanol, and organic acids, e.g. acetic acid. These substances can be used individually or as a mixture.

Nitrogen sources which can be used are organic nitrogen-containing compounds such as peptones, yeast extract, meat extract, malt extract, corn steep liquor, soybean flour and urea, or inorganic compounds such as ammonium sulfate, ammonium chloride, ammonium phosphate, ammonium carbonate and ammonium nitrate. The nitrogen sources can be used individually or as a mixture.

Phosphorus sources which can be used are phosphoric acid, potassium dihydrogenphosphate or dipotassium hydrogenphosphate or the corresponding sodium salts. The culture medium must also contain metal salts, e.g. magnesium sulfate or iron sulfate, which are necessary for growth. Finally, essential growth-promoting substances such as amino acids and vitamins can be used in addition to the substances mentioned above. Suitable precursors can also be added to the culture medium. Said feed materials can be added to the culture all at once or fed in appropriately during cultivation.

The pH of the culture is controlled by the appropriate use of basic compounds such as sodium hydroxide, potassium hydroxide, ammonia or aqueous ammonia, or acidic compounds such as phosphoric acid or sulfuric acid. Foaming can be controlled using antifoams such as fatty acid polyglycol esters. The stability of plasmids can be maintained by adding suitable selectively acting substances, e.g. antibiotics, to the medium. Aerobic conditions are maintained by introducing oxygen or oxygen-containing gaseous mixtures, e.g. air, into the culture. The temperature of the culture is normally 20°C to 45°C and preferably 25°C to 40°C. The culture is continued until the formation of the desired product has reached a maximum. This objective is normally achieved within 10 hours to 160 hours.

Methods of determining L-amino acids are known from the state of the art. They can be analyzed for example by ion exchange chromatography with subsequent ninhydrin derivation, as described by Spackman et al. (Analytical Chemistry 30 (1958) 1190), or by reversed phase HPLC, as described by Lindroth et al. (Analytical Chemistry (1979) 51, 1167-1174).

The fermentation process according to the invention is used for the preparation of L-Lysin.

The present invention is illustrated in greater detail below by means of Examples.

The isolation of plasmid DNA from Escherichia coli and all the techniques of restriction, Klenow treatment and alkaline phosphatase treatment were carried out according to Sambrook et al. (Molecular Cloning. A Laboratory Manual (1989), Cold Spring Harbor Laboratory Press, Cold Spring Harbor, NY, USA). Methods of transforming Escherichia coli are also described in this manual.

The composition of common nutrient media, such as LB or TY medium, can also be found in the manual by Sambrook et al.

### Example 1

### Preparation of a genomic cosmid gene library from Corynebacterium glutamicum ATCC13032

Chromosomal DNA from Corynebacterium glutamicum ATCC13032 was isolated as described in Tauch et al. (1995, Plasmid 33, 168-179) and partially cleaved with the restriction enzyme Sau3AI (Amersham Pharmacia, Freiburg, Germany, product description Sau3AI, code no. 27-0913-02). The DNA fragments were dephosphorylated with shrimp alkaline phosphatase (Roche Diagnostics GmbH, Mannheim, Germany, product description SAP, code no. 1758250). The DNA of cosmid vector SuperCos1 (Wahl et al. (1987), Proceedings of the National Academy of Sciences USA 84, 2160-2164), obtained from Stratagene (La Jolla, USA, product description SuperCos1 Cosmid Vector Kit, code no. 251301), was cleaved with the restriction enzyme XbaI (Amersham Pharmacia, Freiburg, Germany, product description XbaI, code no. 27-0948-02) and also dephosphorylated with shrimp alkaline phosphatase.

The cosmid DNA was then cleaved with the restriction enzyme BamHI (Amersham Pharmacia, Freiburg, Germany, product description BamHI, code no. 27-0868-04). The cosmid DNA treated in this way was mixed with the treated ATCC13032 DNA and the mixture was treated with T4 DNA ligase (Amersham Pharmacia, Freiburg, Germany, product description T4 DNA ligase, code no. 27-0870-04). The ligation mixture was then packaged into phages using Gigapack II XL Packing Extract (Stratagene, La Jolla, USA, product description Gigapack II XL Packing Extract, code no. 200217).

For infection of the E. coli strain NM554 (Raleigh et al., 1988, Nucleic Acid Research 16, 1563-1575), the cells were taken up in 10 mM MgSO₄ and mixed with an aliquot of the phage suspension. Infection and titering of the cosmid library were carried out as described in Sambrook et al. (1989, Molecular Cloning: A Laboratory Manual, Cold Spring Harbor), the cells being plated on LB agar (Lennox, 1955, Virology 1, 190) containing 100 mg/l of ampicillin. After incubation overnight at 37°C, recombinant single clones were selected.

### Example 2

### Isolation and sequencing of the pknB gene

The cosmid DNA of a single colony was isolated with the Qiaprep Spin Miniprep Kit (product no. 27106, Qiagen, Hilden, Germany) in accordance with the manufacturer's instructions and partially cleaved with the restriction enzyme Sau3AI (Amersham Pharmacia, Freiburg, Germany, product description Sau3AI, product no. 27-0913-02). The DNA fragments were dephosphorylated with shrimp alkaline phosphatase (Roche Diagnostics GmbH, Mannheim, Germany, product description SAP, product no. 1758250). After separation by gel electrophoresis, the cosmid fragments in the size range from 1500 to 2000 bp were isolated with the QiaExII Gel Extraction Kit (product no. 20021, Qiagen, Hilden, Germany).

The DNA of sequencing vector pZero-1, obtained from Invitrogen (Groningen, The Netherlands, product description Zero Background Cloning Kit, product no. K2500-01), was cleaved with the restriction enzyme BamHI (Amersham Pharmacia, Freiburg, Germany, product description BamHI, product no. 27-0868-04). Ligation of the cosmid fragments into sequencing vector pZero-1 was carried out as described in Sambrook et al. (1989, Molecular Cloning: A Laboratory Manual, Cold Spring Harbor), the DNA mixture being incubated overnight with T4 ligase (Pharmacia Biotech, Freiburg, Germany). This ligation mixture was then introduced into the E. coli strain DH5αMCR (Grant, 1990, Proceedings of the National Academy of Sciences USA 87, 4645-4649) by electroporation (Tauch et al. 1994, FEMS Microbiol. Letters 123, 343-7) and plated on LB agar (Lennox, 1955, Virology 1, 190) containing 50 mg/l of zeocin.

Plasmid preparation of the recombinant clones was carried out with Biorobot 9600 (product no. 900200, Qiagen, Hilden, Germany). Sequencing was carried out by the dideoxy chain termination method of Sanger et al. (1977, Proceedings of the National Academy of Sciences USA 74, 5463-5467) with modifications by Zimmermann et al. (1990, Nucleic Acids Research 18, 1067). The "RR dRhodamin Terminator Cycle Sequencing Kit" from PE Applied Biosystems (product no. 403044, Weiterstadt, Germany) was used. Separation by gel electrophoresis and analysis of the sequencing reaction were carried out in a "Rotiphorese NF acrylamide/bisacrylamide" gel (29:1) (product no. A124.1, Roth, Karlsruhe, Germany) with the "ABI Prism 377" sequencer from PE Applied Biosystems (Weiterstadt, Germany).

The raw sequence data obtained were then processed using the Staden programming package (1986, Nucleic Acids Research 14, 217-231), version 97-0. The individual sequences of the pZero-1 derivatives were assembled into a cohesive contig. Computer-assisted coding region analysis was performed with the XNIP program (Staden, 1986, Nucleic Acids Research 14, 217-231).

The nucleotide sequence obtained is shown in SEQ ID No. 1.

## Claims

1. An isolated polynucleotide from corynebacteria selected from the group comprising:
a) a polynucleotide containing the nucleotide sequence of SEQ ID NO: 1,
b) a polynucleotide which is complementary to the polynucleotide of a).

2. A polynucleotide according to claim 1 which is a recombinant DNA replicatable in corynebacteria.

3. A polynucleotide according to claim 1 which is an RNA.

4. Corynebacteria in which the pknB gene is overexpressed, wherein said pknB gene is
a) a polynucleotide containg the nucleotide sequence of SEQ ID NO:1.

5. Corynebacteria according to claim 4, wherein overexpression is achieved by increasing the copy number of said polynucleotides or using an inducible promoter.

6. Corynebacteria according to claim 5, wherein increasing the copy number is achieved
a) by transforming corynebacteria with an episomal plasmid; or
b) introducing said polynucleotides into the chromosome.

7. Vector, comprising a polynucleotide according to claims 1 to 3.

8. A fermentation process for the preparation of L-Lysin comprising:
a) fermentation of corynebacteria according to claims 4 to 6, producing the desired L-amino acid.

9. Fermentation process according to claim 8, comprising
a) accumulation of the L-amino acid in the medium or in the cells of the bacteria, and
b) isolation of the L-amino acid.

10. The process according to claims 8 or 9, wherein a strain transformed with a vector is used and the vector carries the nucleotide sequence coding for the pknB gene.

11. The process according to claims 8 or 9, wherein the the polynucleotide(s) coding for the pknB gene product is overexpressed.

12. The process according to one or more of claims 8-11, wherein microorganisms of the species Corynebacterium glutamicum are used.

## Patentansprüche

1. Isoliertes Polynukleotid aus Corynebakterien, ausgewählt aus der Gruppe umfassend:
a) ein Polynukleotid, das die Nukleotidsequenz gemäß SEQ ID NO: 1 enthält,
b) ein Polynukleotid, das zu dem Polynukleotid gemäß a) komplementär ist.

2. Polynukleotid nach Anspruch 1, bei dem es sich um eine rekombinante DNA, die in Corynebakterien replizierbar ist, handelt.

3. Polynukleotid nach Anspruch 1, bei dem es sich um eine RNA handelt.

4. Corynebakterien, in denen das pknB-Gen überexprimiert wird, wobei es sich bei dem pknB-Gen um
a) ein Polynukleotid, enthaltend die Nukleotidsequenz gemäß SEQ ID NO: 1, handelt.

5. Corynebakterien nach Anspruch 4, bei denen die Überexpression dadurch erzielt wird, dass man die Kopienzahl des Polynukleotids erhöht oder einen induzierbaren Promoter verwendet.

6. Corynebakterien nach Anspruch 5, bei denen das Erhöhen der Kopienzahl dadurch erzielt wird, dass man
a) Corynebakterien mit einem episomalen Plasmid transformiert, oder
b) das Polynukleotid in das Chromosom einführt.

7. Vektor, umfassend ein Polynukleotid nach den Ansprüchen 1 bis 3.

8. Fermentationsverfahren zur Herstellung von L-Lysin, das Folgendes umfasst:
a) Fermentieren von Corynebakterien nach den Ansprüchen 4 bis 6, wodurch man die gewünschte L-Aminosäure erhält.

9. Fermentationsverfahren nach Anspruch 8, das Folgendes umfasst:
a) Akkumulieren der L-Aminosäure in dem Medium oder in den Bakterienzellen, und
b) Isolieren der L-Aminosäure.

10. Verfahren nach den Ansprüchen 8 oder 9, wobei man einen mit einem Vektor transformierten Stamm verwendet und der Vektor die für das pknB-Gen kodierende Nukleotidsequenz trägt.

11. Verfahren nach den Ansprüchen 8 oder 9, wobei das/die für das pknB-Genprodukt kodierende(n) Polynukleotid(e) überexprimiert wird/werden.

12. Verfahren nach einem oder mehreren der Ansprüche 8-11, wobei Mikroorganismen der Art Corynebakterium glutamicum verwendet werden.

## Revendications

1. Polynucléotide isolé provenant d'une corynébactérie, choisi dans le groupe comprenant :
a) un polynucléotide contenant la séquence nucléotidique SEQ ID N° 1,
b) un polynucléotide qui est complémentaire du polynucléotide de a).

2. Polynucléotide selon la revendication 1, qui est un ADN recombinant pouvant se répliquer dans une corynébactérie.

3. Polynucléotide selon la revendication 1, qui est un ARN.

4. Corynébactérie dans laquelle le gène pknB est surexprimé, ledit gène pknB étant
a) un polynucléotide contenant la séquence nucléotidique SEQ ID N° 1.

5. Corynébactérie selon la revendication 4, dans laquelle la surexpression est réalisée par augmentation du nombre de copies desdits polynucléotides, ou par utilisation d'un promoteur inductible.

6. Corynébactérie selon la revendication 5, dans laquelle l'augmentation du nombre de copies est réalisée
a) par transformation d'une corynébactérie avec un plasmide épisomique, ou
b) par introduction dudit polynucléotide dans le chromosome.

7. Vecteur comprenant un polynucléotide selon les revendications 1 à 3.

8. Procédé de fermentation pour la préparation de L-lysine, comprenant :
a) la fermentation d'une corynébactérie selon les revendications 4 à 6, pour produire l'acide L-aminé souhaité.

9. Procédé de fermentation selon la revendication 8, comprenant
a) l'accumulation de l'acide L-aminé dans le milieu ou dans les cellules de la bactérie, et
b) l'isolement de l'acide L-aminé.

10. Procédé selon la revendication 8 ou 9, dans lequel on utilise une souche transformée avec un vecteur, le vecteur portant la séquence nucléotidique codant pour le gène pknB.

11. Procédé selon la revendication 8 ou 9, dans lequel le ou les polynucléotides codant pour les produits géniques pknB sont surexprimés.

12. Procédé selon l'une ou plusieurs des revendications 8 à 11, dans lequel on utilise des micro-organismes de l'espèce Corynebacterium glutamicum.


### SEQUENCE LISTING

<110> Degussa AG
<120> Nucleotide sequences coding for the pknB gene
<130> 000505 BT
<140>
   <141>
<160> 4
<170> PatentIn Ver. 2.1
<210> 1
   <211> 2875 <212> DNA
   <213> Corynebacterium glutamicum
<220>
   <221> CDS
   <222> (594)..(2474)
   <223> pknB gene
<400> 1
   cgcacgagcg cgatgatggc gcaggcggag gcgccgtcgc caagcgaatc aacggcgatg 60
   ctgggcaggg tggcccggcc tgcaacaatc acccaagaag cggccccgaa acgcggttcc 120
   ggcattggca ttggtctgtt catcgcagct ttgcttgccg tgattattgg cgcggtgatc 180
   tatgcgggca ccaccggaat tttgttcaac gacactccgg aagaaaccac cacacctgaa 240
   accattacgg aaacatacac cccaaccgtg gaggaaacca cctctcagtg ggtaccgcca 300
   acgcctccaa cacggtcaac attcaccgaa cctgaaacaa cttcacaccg tccgacgaca 360
   agtgaagaga gcacatccga ggaaccaacc acggaagctc caacaagtag ccgaactgtg 420
   cctcaaatcc ctacctctac acctaggacg agtgctagcg ttccagttga gactaatgca 480
   ccggctgatg atttaatcga cgccgtaaat ggcctattgg atgtaggagg agcgcagtga 540
   ccttcgtgat cgctgatcgc tatgaactgg atgccgtcat cggctccggt ggc atg     596
                                                              Met
                                                                1
   agc gag gtg ttc gcg gcc acc gac acg ctc att ggt cgg gag gtc gcg     644
   Ser Glu Val Phe Ala Ala Thr Asp Thr Leu Ile Gly Arg Glu Val Ala
                   5                  10                  15
   gta aag atg ctg cgc atc gac ctt gcg aaa gat ccc aat ttc cga gaa     692
   Val Lys Met Leu Arg Ile Asp Leu Ala Lys Asp Pro Asn Phe Arg Glu
           20                  25                  30
   cgc ttc cgc agg gaa gcc caa aac tcc gga agg ttg agc cac tct tcg     740
   Arg Phe Arg Arg Glu Ala Gln Asn Ser Gly Arg Leu Ser His Ser Ser
       35                  40                  45
   atc gtc gct gtt ttt gac acc ggc gaa gta gac aaa gac ggc acc tct     788
   Ile Val Ala Val Phe Asp Thr Gly Glu Val Asp Lys Asp Gly Thr Ser
    50                  55                  60                  65
   gtt ccc tac att gtg atg gaa cgc gtg cag ggt cga aac ctg cgc gaa     836
   Val Pro Tyr Ile Val Met Glu Arg Val Gln Gly Arg Asn Leu Arg Glu
                    70                  75                  80
   gtt gtc acc gaa gac ggc gta ttc acc cca gtt gag gca gcc aac atc     884
   Val Val Thr Glu Asp Gly Val Phe Thr Pro Val Glu Ala Ala Asn Ile
                85                  90                  95
   ctc atc cct gtg tgt gaa gcg ctg cag gca tcc cat gac gcc ggc att     932
   Leu Ile Pro Val Cys Glu Ala Leu Gln Ala Ser His Asp Ala Gly Ile
           100                 105                 110
   att cac cgc gat gtg aaa ccc gcc aac atc atg atc acc aac acc ggt     980
   Ile His Arg Asp Val Lys Pro Ala Asn Ile Met Ile Thr Asn Thr Gly
       115                 120                 125
   ggc gtg aaa gtc atg gac ttc ggc atc gcc cgc gcg gtc aac gat tcc    1028
   Gly Val Lys Val Met Asp Phe Gly Ile Ala Arg Ala Val Asn Asp Ser
   130                 135                 140                 145
   acc tcc gcc atg act caa acc tcc gca gtc atc ggc acc gcc cag tac    1076
   Thr Ser Ala Met Thr Gln Thr Ser Ala Val Ile Gly Thr Ala Gln Tyr
                   150                 155                 160
   ctc tcc cct gag cag gcc cgc ggc aaa ccc gcc gat gcg cgt tcc gat    1124
   Leu Ser Pro Glu Gln Ala Arg Gly Lys Pro Ala Asp Ala Arg Ser Asp
               165                 170                 175
   att tac gcc acc ggc tgc gtc atg tac gaa tta gtc acc ggt aag cca    1172
   Ile Tyr Ala Thr Gly Cys Val Met Tyr Glu Leu Val Thr Gly Lys Pro
           180                 185                 190
   cct ttt gaa ggc gag tcc cct ttc gcc gtg gcc tac caa cac gtc cag    1220
   Pro Phe Glu Gly Glu Ser Pro Phe Ala Val Ala Tyr Gln His Val Gln
       195                 200                 205
   gaa gac ccc acc cct cct tcg gat ttc atc gcg gac ctc acc ccg acc    1268
   Glu Asp Pro Thr Pro Pro Ser Asp Phe Ile Ala Asp Leu Thr Pro Thr
   210                 215                 220                 225
   tct gct gtc aac gtg gat gcc gtg gta ctc acc gcc atg gca aaa cac    1316
   Ser Ala Val Asn Val Asp Ala Val Val Leu Thr Ala Met Ala Lys His
                   230                 235                 240
   ccc gcc gac cgc tac caa aca gcc tcc gaa atg gcc gct gac ctg ggc    1364
   Pro Ala Asp Arg Tyr Gln Thr Ala Ser Glu Met Ala Ala Asp Leu Gly
               245                 250                 255
   cgg cta tcc cgc aat gca gtc tcc cat gcc gca cgc gcg cat gta gaa    1412
   Arg Leu Ser Arg Asn Ala Val Ser His Ala Ala Arg Ala His Val Glu
           260                 265                 270
   aca gaa gaa acc cca gaa gag ccc gaa act cgc ttc tcg acg cgc acc    1460
   Thr Glu Glu Thr Pro Glu Glu Pro Glu Thr Arg Phe Ser Thr Arg Thr
       275                 280                 285
   tcc acc caa gtg gcc ccc gcc gca ggc gtg gct gcg gcc agt acg ggg    1508
   Ser Thr Gln Val Ala Pro Ala Ala Gly Val Ala Ala Ala Ser Thr Gly
   290                 295                 300                 305
   tca ggg tct tct tcg cgt aaa cgt gga tcc aga ggc ctc acc gcc ctg    1556
   Ser Gly Ser Ser Ser Arg Lys Arg Gly Ser Arg Gly Leu Thr Ala Leu
                   310                 315                 320
   gcc atc gtg tta tcc cta ggt gtc gtc ggc gtt gcc ggt gcc ttc acc    1604
   Ala Ile Val Leu Ser Leu Gly Val Val Gly Val Ala Gly Ala Phe Thr
           325                 330                 335
   tac gac tac ttt gcc aac agc tcc tcc act gca acc agc gcg atc ccc    1652
   Tyr Asp Tyr Phe Ala Asn Ser Ser Ser Thr Ala Thr Ser Ala Ile Pro
       340                 345                 350
   aat gtg gaa ggc ctc ccg cag caa gaa gct ctc aca gaa ctt caa gca    1700
   Asn Val Glu Gly Leu Pro Gln Gln Glu Ala Leu Thr Glu Leu Gln Ala
   355                 360                 365
   gca gga ttt gtt gtc aac atc gtc gaa gaa gcc agc gcc gac gtc gcc    1748
   Ala Gly Phe Val Val Asn Ile Val Glu Glu Ala Ser Ala Asp Val Ala
   370                 375                 380                 385
   gaa ggc ctc gtc atc cga gca aac cca agc gtt gga tcc gaa atc cgc    1796
   Glu Gly Leu Val Ile Arg Ala Asn Pro Ser Val Gly Ser Glu Ile Arg
                   390                 395                 400
   caa ggg gcc acc gtc acc atc acc gtg tcc acc ggc cga gaa atg atc    1844
   Gln Gly Ala Thr Val Thr Ile Thr Val Ser Thr Gly Arg Glu Met Ile
           405                 410                 415
   aac atc cca gac gtc tcc ggc atg aca ctt gag gac gcc gcc cgc gcc    1892
   Asn Ile Pro Asp Val Ser Gly Met Thr Leu Glu Asp Ala Ala Arg Ala
       420                 425                 430
   ctc gaa gac gtt ggt ctc ata ctc aac caa aac gtt cgg gaa gaa acc    1940
   Leu Glu Asp Val Gly Leu Ile Leu Asn Gln Asn Val Arg Glu Glu Thr
   435                 440                 445
   tcc gac gac gtc gaa tct ggc ctc gtc atc gac caa aac ccc gaa gcc    1988
   Ser Asp Asp Val Glu Ser Gly Leu Val Ile Asp Gln Asn Pro Glu Ala
   450                 455                 460                 465
   ggc caa gaa gta gtc gtg ggt tcc tct gta tct cta acc atg tct tca    2036
   Gly Gln Glu Val Val Val Gly Ser Ser Val Ser Leu Thr Met Ser Ser
                   470                 475                 480
   ggc acc gag agc atc cga gtg ccc aac ctc acc ggc atg aac tgg tca    2084
   Gly Thr Glu Ser Ile Arg Val Pro Asn Leu Thr Gly Met Asn Trp Ser
           485                 490                 495
   caa gca gaa caa aac ctc atc tcc atg ggc ttt aac ccc aca gct tcc    2132
   Gln Ala Glu Gln Asn Leu Ile Ser Met Gly Phe Asn Pro Thr Ala Ser
       500                 505                 510
   tac tta gac agc agc gaa cca gaa ggc gaa gtc ctc tca gtt tcc agc    2180
   Tyr Leu Asp Ser Ser Glu Pro Glu Gly Glu Val Leu Ser Val Ser Ser
   515                 520                 525
   caa gga act gaa cta ccc aag ggt tca tcc atc aca gtg gaa gtc tcc   2228
   Gln Gly Thr Glu Leu Pro Lys Gly Ser Ser Ile Thr Val Glu Val Ser
   530                 535                 540                 545
   aac ggc atg ctc atc caa gcc ccc gat ctc gcc cgc atg tcc acc gaa   2276
   Asn Gly Met Leu Ile Gln Ala Pro Asp Leu Ala Arg Met Ser Thr Glu
                   550                 555                 560
   cag gcc atc agt gcc ctc cgc gct gct ggc tgg acc gcc cca gat caa   2324
   Gln Ala Ile Ser Ala Leu Arg Ala Ala Gly Trp Thr Ala Pro Asp Gln
               565                 570                 575
   tcc ctg atc gtc ggc gac ccc atc cac acc gca gcc ctc gtg gat caa   2372
   Ser Leu Ile Val Gly Asp Pro Ile His Thr Ala Ala Leu Val Asp Gln
           580                 585                 590
   aac aaa atc gga ttc caa tcc cca acc cct gca acc ctc ttc cgc aaa   2420
   Asn Lys Ile Gly Phe Gln Ser Pro Thr Pro Ala Thr Leu Phe Arg Lys
       595                 600                 605
   gac gcc caa gtg caa gtg cga ctc ttc gaa ttc gat ctc gct gca ctc   2468
   Asp Ala Gln Val Gln Val Arg Leu Phe Glu Phe Asp Leu Ala Ala Leu
   610                 615                 620                 625
   gtg caa tagccaacaa ggaaaccgtc aaggtagctg gcccggcaac tgatacgtta   2524
   Val Gln
   agctcaaaca agataagtac cagttgctgg ggtttttcca agacaataaa ttatgaaggt 2584
   gtgaacaatg ccaaaggcaa gagtaactaa aaacgagacc gcaccggttt caagcaaccc 2644
   aagcgcaaac cgcaccccgg ttaagatcaa ttccgccgga accccaatgt ggtacaaggt 2704
   catcatgttt gccttcatga tcgtcggcct agcctggttg atcattaact acctcgtggg 2764
   cccacagatc ccattcatgg ctgatcttgg tgcatggaac tatggcatcg gcttcggtct 2824
   gatgatcatc ggcctactca tgaccatggg ttggcgttaa tccttcaaaa a          2875
<210> 2
   <211> 627
   <212> PRT
   <213> Corynebacterium glutamicum
<400> 2
   Met Ser Glu Val Phe Ala Ala Thr Asp Thr Leu Ile Gly Arg Glu Val
   1               5                   10                  15
   Ala Val Lys Met Leu Arg Ile Asp Leu Ala Lys Asp Pro Asn Phe Arg
               20                  25                  30
   Glu Arg Phe Arg Arg Glu Ala Gln Asn Ser Gly Arg Leu Ser His Ser
           35                  40                  45
   Ser Ile Val Ala Val Phe Asp Thr Gly Glu Val Asp Lys Asp Gly Thr
       50                  55                  60
   Ser Val Pro Tyr Ile Val Met Glu Arg Val Gln Gly Arg Asn Leu Arg
   65                  70                  75                  80
   Glu Val Val Thr Glu Asp Gly Val Phe Thr Pro Val Glu Ala Ala Asn
                   85                  90                  95
   Ile Leu Ile Pro Val Cys Glu Ala Leu Gln Ala Ser His Asp Ala Gly
               100                 105                 110
   Ile Ile His Arg Asp Val Lys Pro Ala Asn Ile Met Ile Thr Asn Thr
           115                 120                 125
   Gly Gly Val Lys Val Met Asp Phe Gly Ile Ala Arg Ala Val Asn Asp
       130                 135                 140
   Ser Thr Ser Ala Met Thr Gln Thr Ser Ala Val Ile Gly Thr Ala Gln
   145                 150                 155                 160
   Tyr Leu Ser Pro Glu Gln Ala Arg Gly Lys Pro Ala Asp Ala Arg Ser
                   165                 170                 175
   Asp Ile Tyr Ala Thr Gly Cys Val Met Tyr Glu Leu Val Thr Gly Lys
               180                 185                 190
   Pro Pro Phe Glu Gly Glu Ser Pro Phe Ala Val Ala Tyr Gln His Val
           195                 200                 205
   Gln Glu Asp Pro Thr Pro Pro Ser Asp Phe Ile Ala Asp Leu Thr Pro
       210                 215                 220
   Thr Ser Ala Val Asn Val Asp Ala Val Val Leu Thr Ala Met Ala Lys
   225                 230                 235                 240
   His Pro Ala Asp Arg Tyr Gln Thr Ala Ser Glu Met Ala Ala Asp Leu
                   245                 250                 255
   Gly Arg Leu Ser Arg Asn Ala Val Ser His Ala Ala Arg Ala His Val
               260                 265                 270
   Glu Thr Glu Glu Thr Pro Glu Glu Pro Glu Thr Arg Phe Ser Thr Arg
           275                 280                 285
   Thr Ser Thr Gln Val Ala Pro Ala Ala Gly Val Ala Ala Ala Ser Thr
       290                 295                 300
   Gly Ser Gly Ser Ser Ser Arg Lys Arg Gly Ser Arg Gly Leu Thr Ala
   305                 310                 315                 320
   Leu Ala Ile Val Leu Ser Leu Gly Val Val Gly Val Ala Gly Ala Phe
                   325                 330                 335
   Thr Tyr Asp Tyr Phe Ala Asn Ser Ser Ser Thr Ala Thr Ser Ala Ile
               340                 345                 350
   Pro Asn Val Glu Gly Leu Pro Gln Gln Glu Ala Leu Thr Glu Leu Gln
           355                 360                 365
   Ala Ala Gly Phe Val Val Asn Ile Val Glu Glu Ala Ser Ala Asp Val
       370                 375                 380
   Ala Glu Gly Leu Val Ile Arg Ala Asn Pro Ser Val Gly Ser Glu Ile
   385 390 395 400
   Arg Gln Gly Ala Thr Val Thr Ile Thr Val Ser Thr Gly Arg Glu Met
   405 410 415
   Ile Asn Ile Pro Asp Val Ser Gly Met Thr Leu Glu Asp Ala Ala Arg
   420 425 430
   Ala Leu Glu Asp Val Gly Leu Ile Leu Asn Gln Asn Val Arg Glu Glu
   435 440 445
   Thr Ser Asp Asp Val Glu Ser Gly Leu Val Ile Asp Gln Asn Pro Glu
   450 455 460
   Ala Gly Gln Glu Val Val Val Gly Ser Ser Val Ser Leu Thr Met Ser
   465 470 475 480
   Ser Gly Thr Glu Ser Ile Arg Val Pro Asn Leu Thr Gly Met Asn Trp
   485 490 495
   Ser Gln Ala Glu Gln Asn Leu Ile Ser Met Gly Phe Asn Pro Thr Ala
   500 505 510
   Ser Tyr Leu Asp Ser Ser Glu Pro Glu Gly Glu Val Leu Ser Val Ser
   515 520 525
   Ser Gln Gly Thr Glu Leu Pro Lys Gly Ser Ser Ile Thr Val Glu Val
   530 535 540
   Ser Asn Gly Met Leu Ile Gln Ala Pro Asp Leu Ala Arg Met Ser Thr
   545 550 555 560
   Glu Gln Ala Ile Ser Ala Leu Arg Ala Ala Gly Trp Thr Ala Pro Asp
   565 570 575
   Gln Ser Leu Ile Val Gly Asp Pro Ile His Thr Ala Ala Leu Val Asp
   580 585 590
   Gln Asn Lys Ile Gly Phe Gln Ser Pro Thr Pro Ala Thr Leu Phe Arg
   595 600 605
   Lys Asp Ala Gln Val Gln Val Arg Leu Phe Glu Phe Asp Leu Ala Ala
   610 615 620
   Leu Val Gln
   625
<210> 3
   <211> 2875
   <212> DNA
   <213> Corynebacterium glutamicum
<220>
   <221> CDS
   <222> (594)..(2474)
<223> pknB-1547 allele
<220>
   <221> misc_feature
   <222> (2343)
   <223> C-T Transition
<400> 3
   cgcacgagcg cgatgatggc gcaggcggag gcgccgtcgc caagcgaatc aacggcgatg 60
   ctgggcaggg tggcccggcc tgcaacaatc acccaagaag cggccccgaa acgcggttcc 120
   ggcattggca ttggtctgtt catcgcagct ttgcttgccg tgattattgg cgcggtgatc 180
   tatgcgggca ccaccggaat tttgttcaac gacactccgg aagaaaccac cacacctgaa 240
   accattacgg aaacatacac cccaaccgtg gaggaaacca cctctcagtg ggtaccgcca 300
   acgcctccaa cacggtcaac attcaccgaa cctgaaacaa cttcacaccg tccgacgaca 360
   agtgaagaga gcacatccga ggaaccaacc acggaagctc caacaagtag ccgaactgtg 420
   cctcaaatcc ctacctctac acctaggacg agtgctagcg ttccagttga gactaatgca 480
   ccggctgatg atttaatcga cgccgtaaat ggcctattgg atgtaggagg agcgcagtga 540
   ccttcgtgat cgctgatcgc tatgaactgg atgccgtcat cggctccggt ggc atg    596
                                                              Met
                                                              1
   agc gag gtg ttc gcg gcc acc gac acg ctc att ggt cgg gag gtc gcg   644
   Ser Glu Val Phe Ala Ala Thr Asp Thr Leu Ile Gly Arg Glu Val Ala
               5                   10                  15
   gta aag atg ctg cgc atc gac ctt gcg aaa gat ccc aat ttc cga gaa   692
   Val Lys Met Leu Arg Ile Asp Leu Ala Lys Asp Pro Asn Phe Arg Glu
           20                  25                  30
   cgc ttc cgc agg gaa gcc caa aac tcc gga agg ttg agc cac tct tcg   740
   Arg Phe Arg Arg Glu Ala Gln Asn Ser Gly Arg Leu Ser His Ser Ser
       35                  40                  45
   atc gtc gct gtt ttt gac acc ggc gaa gta gac aaa gac ggc acc tct   788
   Ile Val Ala Val Phe Asp Thr Gly Glu Val Asp Lys Asp Gly Thr Ser
   50                  55                  60                  65
   gtt ccc tac att gtg atg gaa cgc gtg cag ggt cga aac ctg cgc gaa   836
   Val Pro Tyr Ile Val Met Glu Arg Val Gln Gly Arg Asn Leu Arg Glu
                   70                  75                  80
   gtt gtc acc gaa gac ggc gta ttc acc cca gtt gag gca gcc aac atc   884
   Val Val Thr Glu Asp Gly Val Phe Thr Pro Val Glu Ala Ala Asn Ile
               85                  90                  95
   ctc atc cct gtg tgt gaa gcg ctg cag gca tcc cat gac gcc ggc att   932
   Leu Ile Pro Val Cys Glu Ala Leu Gln Ala Ser His Asp Ala Gly Ile
           100                 105                 110
   att cac cgc gat gtg aaa ccc gcc aac atc atg atc acc aac acc ggt    980
   Ile His Arg Asp Val Lys Pro Ala Asn Ile Met Ile Thr Asn Thr Gly
       115                 120                 125
   ggc gtg aaa gtc atg gac ttc ggc atc gcc cgc gcg gtc aac gat tcc    1028
   Gly Val Lys Val Met Asp Phe Gly Ile Ala Arg Ala Val Asn Asp Ser
   130                 135                 140                 145
   acc tcc gcc atg act caa acc tcc gca gtc atc ggc acc gcc cag tac    1076
   Thr Ser Ala Met Thr Gln Thr Ser Ala Val Ile Gly Thr Ala Gln Tyr
                   150                 155                 160
   ctc tcc cct gag cag gcc cgc ggc aaa ccc gcc gat gcg cgt tcc gat    1124
   Leu Ser Pro Glu Gln Ala Arg Gly Lys Pro Ala Asp Ala Arg Ser Asp
               165                 170                 175
   att tac gcc acc ggc tgc gtc atg tac gaa tta gtc acc ggt aag cca    1172
   Ile Tyr Ala Thr Gly Cys Val Met Tyr Glu Leu Val Thr Gly Lys Pro
           180                 185                 190
   cct ttt gaa ggc gag tcc cct ttc gcc gtg gcc tac caa cac gtc cag    1220
   Pro Phe Glu Gly Glu Ser Pro Phe Ala Val Ala Tyr Gln His Val Gln
       195                 200                 205
   gaa gac ccc acc cct cct tcg gat ttc atc gcg gac ctc acc ccg acc    1268
   Glu Asp Pro Thr Pro Pro Ser Asp Phe Ile Ala Asp Leu Thr Pro Thr
   210                 215                 220                 225
   tct gct gtc aac gtg gat gcc gtg gta ctc acc gcc atg gca aaa cac    1316
   Ser Ala Val Asn Val Asp Ala Val Val Leu Thr Ala Met Ala Lys His
                   230                 235                 240
   ccc gcc gac cgc tac caa aca gcc tcc gaa atg gcc gct gac ctg ggc    1364
   Pro Ala Asp Arg Tyr Gln Thr Ala Ser Glu Met Ala Ala Asp Leu Gly
               245                 250                 255
   cgg cta tcc cgc aat gca gtc tcc cat gcc gca cgc gcg cat gta gaa    1412
   Arg Leu Ser Arg Asn Ala Val Ser His Ala Ala Arg Ala His Val Glu
           260                 265                 270
   aca gaa gaa acc cca gaa gag ccc gaa act cgc ttc tcg acg cgc acc    1460
   Thr Glu Glu Thr Pro Glu Glu Pro Glu Thr Arg Phe Ser Thr Arg Thr
       275                 280                 285
   tcc acc caa gtg gcc ccc gcc gca ggc gtg gct gcg gcc agt acg ggg    1508
   Ser Thr Gln Val Ala Pro Ala Ala Gly Val Ala Ala Ala Ser Thr Gly
   290                 295                 300                 305
   tca ggg tct tct tcg cgt aaa cgt gga tcc aga ggc ctc acc gcc ctg    1556
   Ser Gly Ser Ser Ser Arg Lys Arg Gly Ser Arg Gly Leu Thr Ala Leu
                   310                 315                 320
   gcc atc gtg tta tcc cta ggt gtc gtc ggc gtt gcc ggt gcc ttc acc    1604
   Ala Ile Val Leu Ser Leu Gly Val Val Gly Val Ala Gly Ala Phe Thr
               325                 330                 335
   tac gac tac ttt gcc aac agc tcc tcc act gca acc agc gcg atc ccc    1652
   Tyr Asp Tyr Phe Ala Asn Ser Ser Ser Thr Ala Thr Ser Ala Ile Pro
           340                 345                 350
   aat gtg gaa ggc ctc ccg cag caa gaa gct ctc aca gaa ctt caa gca 1700
   Asn Val Glu Gly Leu Pro Gln Gln Glu Ala Leu Thr Glu Leu Gln Ala
   355 360 365
   gca gga ttt gtt gtc aac atc gtc gaa gaa gcc agc gcc gac gtc gcc 1748
   Ala Gly Phe Val Val Asn Ile Val Glu Glu Ala Ser Ala Asp Val Ala
   370 375 380 385
   gaa ggc ctc gtc atc cga gca aac cca agc gtt gga tcc gaa atc cgc 1796
   Glu Gly Leu Val Ile Arg Ala Asn Pro Ser Val Gly Ser Glu Ile Arg
   390 395 400
   caa ggg gcc acc gtc acc atc acc gtg tcc acc ggc cga gaa atg atc 1844
   Gln Gly Ala Thr Val Thr Ile Thr Val Ser Thr Gly Arg Glu Met Ile
   405 410 415
   aac atc cca gac gtc tcc ggc atg aca ctt gag gac gcc gcc cgc gcc 1892
   Asn Ile Pro Asp Val Ser Gly Met Thr Leu Glu Asp Ala Ala Arg Ala
   420 425 430
   ctc gaa gac gtt ggt ctc ata ctc aac caa aac gtt cgg gaa gaa acc 1940
   Leu Glu Asp Val Gly Leu Ile Leu Asn Gln Asn Val Arg Glu Glu Thr
   435 440 445
   tcc gac gac gtc gaa tct ggc ctc gtc atc gac caa aac ccc gaa gcc 1988
   Ser Asp Asp Val Glu Ser Gly Leu Val Ile Asp Gln Asn Pro Glu Ala
   450 455 460 465
   ggc caa gaa gta gtc gtg ggt tcc tct gta tct cta acc atg tct tca 2036
   Gly Gln Glu Val Val Val Gly Ser Ser Val Ser Leu Thr Met Ser Ser
   470 475 480
   ggc acc gag agc atc cga gtg ccc aac ctc acc ggc atg aac tgg tca 2084
   Gly Thr Glu Ser Ile Arg Val Pro Asn Leu Thr Gly Met Asn Trp Ser
   485 490 495
   caa gca gaa caa aac ctc atc tcc atg ggc ttt aac ccc aca gct tcc 2132
   Gln Ala Glu Gln Asn Leu Ile Ser Met Gly Phe Asn Pro Thr Ala Ser
   500 505 510
   tac tta gac agc agc gaa cca gaa ggc gaa gtc ctc tca gtt tcc agc 2180
   Tyr Leu Asp Ser Ser Glu Pro Glu Gly Glu Val Leu Ser Val Ser Ser
   515 520 525
   caa gga act gaa cta ccc aag ggt tca tcc atc aca gtg gaa gtc tcc 2228
   Gln Gly Thr Glu Leu Pro Lys Gly Ser Ser Ile Thr Val Glu Val Ser
   530 535 540 545
   aac ggc atg ctc atc caa gcc ccc gat ctc gcc cgc atg tcc acc gaa 2276
   Asn Gly Met Leu Ile Gln Ala Pro Asp Leu Ala Arg Met Ser Thr Glu
   550 555 560
   cag gcc atc agt gcc ctc cgc gct gct ggc tgg acc gcc cca gat caa 2324
   Gln Ala Ile Ser Ala Leu Arg Ala Ala Gly Trp Thr Ala Pro Asp Gln
   565 570 575
   tcc ctg atc gtc ggc gac tcc atc cac acc gca gcc ctc gtg gat caa 2372
   Ser Leu Ile Val Gly Asp Ser Ile His Thr Ala Ala Leu Val Asp Gln
   580 585 590
   aac aaa atc gga ttc caa tcc cca acc cct gca acc ctc ttc cgc aaa    2420
   Asn Lys Ile Gly Phe Gln Ser Pro Thr Pro Ala Thr Leu Phe Arg Lys
       595                 600                 605
   gac gcc caa gtg caa gtg cga ctc ttc gaa ttc gat ctc gct gca ctc    2468
   Asp Ala Gln Val Gln Val Arg Leu Phe Glu Phe Asp Leu Ala Ala Leu
   610                 615                 620                 625
   gtg caa tagccaacaa ggaaaccgtc aaggtagctg gcccggcaac tgatacgtta     2524
   Val Gln
   agctcaaaca agataagtac cagttgctgg ggtttttcca agacaataaa ttatgaaggt 2584
   gtgaacaatg ccaaaggcaa gagtaactaa aaacgagacc gcaccggttt caagcaaccc 2644
   aagcgcaaac cgcaccccgg ttaagatcaa ttccgccgga accccaatgt ggtacaaggt 2704
   catcatgttt gccttcatga tcgtcggcct agcctggttg atcattaact acctcgtggg 2764
   cccacagatc ccattcatgg ctgatcttgg tgcatggaac tatggcatcg gcttcggtct 2824
   gatgatcatc ggcctactca tgaccatggg ttggcgttaa tccttcaaaa a          2875
<210> 4
   <211> 627
   <212> PRT
   <213> Corynobacrterium glutamicum
<400> 4
   Met Ser Glu Val Phe Ala Ala Thr Asp Thr Leu Ile Gly Arg Glu Val
   1               5                   10                  15
   Ala Val Lys Met Leu Arg Ile Asp Leu Ala Lys Asp Pro Asn Phe Arg
               20                  25                  30
   Glu Arg Phe Arg Arg Glu Ala Gln Asn Ser Gly Arg Leu Ser His Ser
           35                  40                  45
   Ser Ile Val Ala Val Phe Asp Thr Gly Glu Val Asp Lys Asp Gly Thr
       50                  55                  60
   Ser Val Pro Tyr Ile Val Met Glu Arg Val Gln Gly Arg Asn Leu Arg
   65                  70                  75                  80
   Glu Val Val Thr Glu Asp Gly Val Phe Thr Pro Val Glu Ala Ala Asn
                   85                  90                  95
   Ile Leu Ile Pro Val Cys Glu Ala Leu Gln Ala Ser His Asp Ala Gly
               100                 105                 110
   Ile Ile His Arg Asp Val Lys Pro Ala Asn Ile Met Ile Thr Asn Thr
           115                 120                 125
   Gly Gly Val Lys Val Met Asp Phe Gly Ile Ala Arg Ala Val Asn Asp
       130                 135                 140
   Ser Thr Ser Ala Met Thr Gln Thr Ser Ala Val Ile Gly Thr Ala Gln
   145 150 155 160
   Tyr Leu Ser Pro Glu Gln Ala Arg Gly Lys Pro Ala Asp Ala Arg Ser
   165 170 175
   Asp Ile Tyr Ala Thr Gly Cys Val Met Tyr Glu Leu Val Thr Gly Lys
   180 185 190
   Pro Pro Phe Glu Gly Glu Ser Pro Phe Ala Val Ala Tyr Gln His Val
   195 200 205
   Gln Glu Asp Pro Thr Pro Pro Ser Asp Phe Ile Ala Asp Leu Thr Pro
   210 215 220
   Thr Ser Ala Val Asn Val Asp Ala Val Val Leu Thr Ala Met Ala Lys
   225 230 235 240
   His Pro Ala Asp Arg Tyr Gln Thr Ala Ser Glu Met Ala Ala Asp Leu
   245 250 255
   Gly Arg Leu Ser Arg Asn Ala Val Ser His Ala Ala Arg Ala His Val
   260 265 270
   Glu Thr Glu Glu Thr Pro Glu Glu Pro Glu Thr Arg Phe Ser Thr Arg
   275 280 285
   Thr Ser Thr Gln Val Ala Pro Ala Ala Gly Val Ala Ala Ala Ser Thr
   290 295 300
   Gly Ser Gly Ser Ser Ser Arg Lys Arg Gly Ser Arg Gly Leu Thr Ala
   305 310 315 320
   Leu Ala Ile Val Leu Ser Leu Gly Val Val Gly Val Ala Gly Ala Phe
   325 330 335
   Thr Tyr Asp Tyr Phe Ala Asn Ser Ser Ser Thr Ala Thr Ser Ala Ile
   340 345 350
   Pro Asn Val Glu Gly Leu Pro Gln Gln Glu Ala Leu Thr Glu Leu Gln
   355 360 365
   Ala Ala Gly Phe Val Val Asn Ile Val Glu Glu Ala Ser Ala Asp Val
   370 375 380
   Ala Glu Gly Leu Val Ile Arg Ala Asn Pro Ser Val Gly Ser Glu Ile
   385 390 395 400
   Arg Gln Gly Ala Thr Val Thr Ile Thr Val Ser Thr Gly Arg Glu Met
   405 410 415
   Ile Asn Ile Pro Asp Val Ser Gly Met Thr Leu Glu Asp Ala Ala Arg
   420 425 430
   Ala Leu Glu Asp Val Gly Leu Ile Leu Asn Gln Asn Val Arg Glu Glu
   435 440 445
   Thr Ser Asp Asp Val Glu Ser Gly Leu Val Ile Asp Gln Asn Pro Glu
   450 455 460
   Ala Gly Gln Glu Val Val Val Gly Ser Ser Val Ser Leu Thr Met Ser
   465 470 475 480
   Ser Gly Thr Glu Ser Ile Arg Val Pro Asn Leu Thr Gly Met Asn Trp
   485 490 495
   Ser Gln Ala Glu Gln Asn Leu Ile Ser Met Gly Phe Asn Pro Thr Ala
   500 505 510
   Ser Tyr Leu Asp Ser Ser Glu Pro Glu Gly Glu Val Leu Ser Val Ser
   515 520 525
   Ser Gln Gly Thr Glu Leu Pro Lys Gly Ser Ser Ile Thr Val Glu Val
   530 535 540
   Ser Asn Gly Met Leu Ile Gln Ala Pro Asp Leu Ala Arg Met Ser Thr
   545 550 555 560
   Glu Gln Ala Ile Ser Ala Leu Arg Ala Ala Gly Trp Thr Ala Pro Asp
   565 570 575
   Gln Ser Leu Ile Val Gly Asp Ser Ile His Thr Ala Ala Leu Val Asp
   580 585 590
   Gln Asn Lys Ile Gly Phe Gln Ser Pro Thr Pro Ala Thr Leu Phe Arg
   595 600 605
   Lys Asp Ala Gln Val Gln Val Arg Leu Phe Glu Phe Asp Leu Ala Ala
   610 615 620
   Leu Val Gln
   625